Europäisches Patentamt

European Patent Office

Office européen des brevets

(11) Numéro de publication : **0 659 881 A1**

(12) **DEMANDE DE BREVET EUROPEEN**

(21) Numéro de dépôt : **94402996.6**

(22) Date de dépôt : **22.12.94**

(51) Int. Cl.$^6$ : **C12N 15/10,** C12N 15/11, C12Q 1/68, C12Q 1/70

(30) Priorité : **22.12.93 FR 9315504**

(43) Date de publication de la demande : **28.06.95 Bulletin 95/26**

(84) Etats contractants désignés : **BE CH DE DK ES FR GB IT LI NL PT SE**

(71) Demandeur : **BIO MERIEUX F-69280 Marcy l'Etoile (FR)**

(72) Inventeur : **Mallet, François 84, rue Anatole France F-69100 Villeurbanne (FR)** Inventeur : **Guillou-Bonnici, Françoise 14, rue Dedieu F-69100 Villeurbanne (FR)** Inventeur : **Cleuziat, Philippe 25, Place des Pavillons F-69007 Lyon (FR)** Inventeur : **Levasseur, Pierre 54, rue Auguste Comte F-69002 Lyon (FR)**

(74) Mandataire : **Tonnellier, Jean-Claude Cabinet Nony & Cie. 29, rue Cambacérès F-75008 Paris (FR)**

(54) **Promoteur modifié pour ARN polymérase, sa préparation et ses applications.**

(57)    Oligonucléotide utilisable comme brin non-matrice de promoteur, dans la transcription d'une séquence d'une cible nucléotidique, en présence d'une ARN polymérase phagique choisie parmi celles des phages dont les ARN polymérases ont des promoteurs spécifiques comprenant une séquence consensus au moins depuis la position -17 jusqu'à la position -1, caractérisé par le fait :

— qu'il comprend une séquence de coeur flanquée à l'une au moins de ses extrémités d'une séquence nucléotidique capable d'hybridation avec une séquence de la cible,

— que ladite séquence de coeur est constituée par une séquence de 6 à 9 nucléotides consécutifs choisis dans la région -12 à -4 du brin non-matrice dudit promoteur spécifique, ou une séquence suffisamment homologue permettant de conserver la fonctionnalité de ladite ARN polymérase,

— qu'une séquence flanquante est complémentaire d'une première région de la cible, et que l'autre séquence flanquante, lorsqu'elle est présente, est complémentaires d'une seconde région de la cible, lesdites première et seconde régions étant séparées, sur la cible, par une séquence ayant un nombre de nucléotides égal au nombre de nucléotides de la séquence de coeur,

— et que le nombre de nucléotides de la région flanquante, ou la somme du nombre de nucléotides des régions flanquantes, est au moins suffisamment élevé pour que ledit oligonucléotide puisse s'hybrider avec la cible à la température d'utilisation de l'ARN polymérase.

Un tel oligonucléotide permet d'initier la transcription en un site de la cible qui n'est normalement pas un site d'initiation de la transcription pour ladite ARN polymérase.

**FIGURE 6**

Promoteur phagique

Hémipromoteur

ADN cible

ARN polymérase phagique +rNTPs

ARN

La présente invention a pour objet un promoteur modifié pour ARN polymérase et ses applications. Plus particulièrement, elle concerne l'utilisation d'un promoteur pour les ARN polymérases phagiques, ce promoteur étant modifié par rapport aux promoteurs présents dans la nature ou déjà décrits. On a découvert qu'il est possible de réduire la taille de la séquence promotrice et d'intégrer des mésappariements entre les deux brins du promoteur. Les promoteurs modifiés obtenus selon l'invention permettent notamment d'effectuer la transcription d'une cible nucléotidique à partir d'un site qui n'est normalement pas un site d'initiation de la transcription pour l'ARN polymérase utilisée. Grâce à l'invention, il devient possible de transcrire *in vitro* des séquences très diverses qui ne sont pas transcrites par les ARN polymérases phagiques lorsqu'on utilise les promoteurs de type sauvage (c'est-à-dire les promoteurs naturels) desdites ARN polymérases. L'utilisation de ces promoteurs modifiés permet de plus d'obtenir différentes efficacités d'initiation de la transcription pour la transcription différentielle de plusieurs séquences au sein d'un même milieu réactionnel.

On sait que l'ARN (acide ribonucléique) est le produit de transcription d'une molécule d'ADN (acide désoxyribonucléique) synthétisé sous l'action d'une enzyme, l'ARN polymérase ADN dépendante.

Il est intéressant à plusieurs titres de pouvoir, à partir d'une séquence d'ADN, obtenir plusieurs séquences d'ARN. Différentes applications de l'obtention de séquences d'ARN spécifiques ont déjà décrites, comme par exemple la synthèse de sondes ARN, ou d'oligoribonucléotides (voir notamment Milligan, J. F., Groebe, D. R., Witherell, G. W., and Uhlenbeck, O. C. (1987) *Nucleic Acids Res.* **25**, 8783-8798), ou l'expression de gènes (voir notamment Steen, R. et al. (1986) *EMBO J.* **5**, 1099-1103 et Fuerst, T. R et al. (1987) *Molecular and Cellular Probes* **7**, 2538-2544 et les demandes de brevet WO 91 05 866 et EP 0178 863), ou encore l'amplification de gène comme décrit par Kievits, T. et al. (*Journal of Virological Methods* **35**, 273-286 (1991)) et Kwoh, D. Y. et al (*Proc. Natl. Acad. Sci. USA* **86**, 1173-1177 (1989)) ou dans les demandes de brevet WO 88 10315 et WO 91 02818.

L'une des caractéristiques propres aux ARN polymérases ADN dépendantes est d'initier la synthèse d'ARN suivant une matrice ADN à partir d'un site particulier d'initiation grâce à la reconnaissance d'une séquence d'acide nucléique, appelée promoteur, qui permet de définir la localisation précise et le brin sur lequel l'initiation doit être effectuée. Contrairement aux ADN polymérases ADN dépendantes, la polymérisation par les ARN polymérases ADN dépendantes n'est pas initiée à partir d'une extrémité 3'OH, et leur substrat naturel est un double brin d'ADN intact.

On définit ci-après quelques termes génériques qui vont être employés.

Par oligonucléotide ou polynucléotide, on entend des séquences nucléotidiques contenant les bases naturelles (A, C, G, T, U) et/ou une ou plusieurs bases modifiées, telles que l'inosine, la méthyl-5-désoxycytidine, la désoxyuridine, la diméthylamino-5-désoxyuridine, la diamino-2,6-purine, la bromo-5-désoxyuridine ou toutes bases modifiées, permettant l'hybridation, en particulier par toutes modifications chimiques appropriées permettant d'accroître le rendement d'hybridation. Des exemples de telles modifications sont notamment l'introduction entre au moins deux nucléotides d'un groupement choisi parmi les esters diphosphates d'alkyl- et/ou d'aryl-phosphonates et/ou phosphorothioates, ou le remplacement d'au moins un sucre (ribose ou désoxyribose) par un polyamide voir par exemple Nielsen P.E. et al., *Science*, 254, 1497-1500 (1991). L'expression "nucléotide" ou "base" désigne non seulement un désoxyribonucléotide ou ribonucléotide naturel, mais tout nucléotide modifié comme il vient d'être dit.

Par promoteur, on entend toute séquence d'acide nucléique capable d'être reconnue par une ARN polymérase pour initier la transcription, c'est-à-dire la synthèse d'un ARN. Cette ARN polymérase peut être soit ADN dépendante, soit ARN dépendante.

Par promoteur naturel, on entend les séquences de promoteur, présentes dans le génome, codant pour l'ARN polymérase dont ce promoteur est spécifique. Par séquence promotrice on entend la séquence d'un oligonucléotide qui entre dans la composition de l'un des brins du promoteur.

Par transcription, on entend la néosynthèse de plusieurs brins d'ARN complémentaires de la séquence (ou cible) se trouvant sous le contrôle du promoteur, le promoteur permettant l'initiation de cette réaction. Cette séquence se trouve en général contiguë à l'extrémité 5' du brin matrice du promoteur. Les brins d'ARN synthétisés sont appelés transcrits.

Par transcription différentielle, on entend une transcription donnant des nombres différents de transcrits à partir de matrices différentes.

Par mutation, on entend tout changement de séquence par rapport à la séquence naturelle. La mutation peut concerner les deux bases complémentaires positionnées sur chaque brin d'un duplex d'acide nucléique, ou n'en concerner qu'une. Si la mutation concerne une seule des deux bases complémentaires, elle donne lieu à l'apparition de ce qu'on appelle un mésappariement.

Par mésappariement, on entend l'introduction à l'intérieur d'un double brin d'acide nucléique de paires de bases autre que A:T, C:G ou A:U.

Par non-appariement on entend la présence de nucléotides qui ne sont pas appariés avec une base du brin d'acide nucléique complémentaire, de par leur nature, ou de par leur position dans la séquence. Si plusieurs bases d'un même brin sont non appariées, elles peuvent former des liaisons entre elles, qui peuvent provoquer l'apparition de structures secondaires dites "boucles" ou "tiges-boucles".

Par délétion, on entend l'élimination de certains nucléotides d'une séquence. Une délétion peut concerner une seule base, ou plusieurs bases contiguës. Si plusieurs bases non contiguës sont concernées, on parlera de délétions multiples. Sur un acide nucléique double brin, une délétion concerne en général à la fois les deux bases complémentaires positionnées sur chaque brin d'acide nucléique. Cependant, une délétion peut aussi intervenir seulement sur une ou plusieurs bases d'un seul brin, et provoquer des non-appariements.

Par séquence consensus, on entend une séquence nucléotidique théorique dans laquelle le nucléotide, à chaque site, est celui qui apparaît le plus fréquemment à ce site, dans les différentes formes naturelles de l'élément génétique considéré (par exemple le promoteur). Le terme "consensus" désigne également toute séquence réelle très proche de la séquence consensus théorique.

On rappelle que la transcription s'effectue par synthèse, dans le sens 5'→3', d'un ARN antiparallèle et complémentaire du brin nucléotidique transcrit (appelé "brin matrice", ou encore "brin antisens"). Le brin d'ADN qui est complémentaire du brin transcrit est appelé "brin non-matrice" ou "brin sens". Par convention, le nucléotide à partir duquel la transcription commence est noté +1 (ou simplement 1). Sur le brin matrice, les nucléotides successifs situés du côté de l'extrémité 3' (région amont) sont, à partir de +1, numérotés -1, -2, etc. Une région aval, par rapport à un nucléotide donné (ou à une séquence donnée) est située vers l'extrémité 5' du brin matrice, et donc vers l'extrémité 3' du brin d'ARN synthétisé. A partir de +1, les nucléotides en aval correspondent successivement aux positions +2, +3, etc.

Comparées aux ARN polymérases bactériennes, eucaryotes, ou mitochondriales, les ARN polymérases phagiques sont des enzymes très simples. Parmi celles-ci, les mieux connues sont les ARN polymérases des bactériophages T7, T3 et SP6. L'ARN polymérase du bactériophage T7 est clonée (voir notamment le brevet US 4952496). Ces enzymes sont très homologues entre elles et sont formées par une seule sous-unité de 98 à 100 kDa. Deux autres polymérases phagiques partagent ces homologies celle du phage K11 de Klebsielle et celle du phage BA14 ; voir Diaz et al., J. Mol. Biol. 229 : 805-811 (1993).

Les promoteurs naturels spécifiques des ARN polymérases des phages T7, T3 et SP6 sont bien connus. Le séquençage du génome entier du bactériophage T7 en 1983 par Dunn et al (*J. Mol. Biol.* **166**, 477-535 (1983)) a permis de définir l'existence de 17 promoteurs sur cet ADN représentés dans le tableau 1 ci-dessous (brin non-matrice).

## Tableau 1

*Promoteurs pour la T7 ARN polymérase*

| Promoteur | | Nucléotide | | |
|---|---|---|---|---|
| | | -10 | 1 | |
| Séquence conservée | | TAATACGACTCACTATAGGGAGA | | |
| Promoteur de réplication | -20 | -10 | 1 | |
| øOL | TTGTCTTTAT | TAATACAACTCACTATAAGGAGA | GA | |
| Promoteurs de classe II | -20 | -10 | 1 | |
| ø1-1A | AACGCCAAAT | CAATACGACTCACTATAGAGGGA | CA | |
| ø1-1B | TTCTTCCGGT | TAATACGACTCACTATAGGAGGA | CC | |
| ø1-3 | GGACTGGAAG | TAATACGACTCAGTATAGGGACA | AT | |
| ø1-5 | AGTTAACTGG | TAATACGACTCACTAAAGGAGGT | AC | |
| ø1-6 | TGGTCACGCT | TAATACGACTCACTAAAGGAGAC | AC | |
| ø2-5 | AGCACCGAAG | TAATACGACTCACTATTAGGGAA | GA | |
| ø3-8 | CGTGGATAAT | TAATTGAACTCACTATTAGGGAA | GA | |
| ø4c | CCGACTGAGA | CAATCCGACTCACTAAAGAGAGA | GA | |
| ø4-3 | AGTCCCATTC | TAATACGACTCACTAAAGGAGAG | AC | |
| ø4-7 | TTCATGAATA | CTATTCGACTCACTATAGGAGAT | AT | |
| Promoteurs de classe III | -20 | -10 | 1 | |
| ø6-5 | GTCCCTAAAT | TAATACGACTCACTATAGGGAGA | TA | |
| ø9 | GCCGGGAATT | TAATACGACTCACTATAGGGAGA | CC | |
| ø10 | ACTTCGAAAT | TAATACGACTCACTATAGGGAGA | CC | |
| ø13 | GGCTCGAAAT | TAATACGACTCACTATAGGGAGA | AC | |
| ø17 | GCGTACCAAA | TAATACGACTCACTATAGGGAGA | GG | |
| Promoteur de réplication | -20 | -10 | 1 | |
| øOR | CACGATAAAT | TAATACGACTCACTATAGGGAGA | GG | |

Comme cela ressort du tableau ci-dessus, vingt trois nucléotides contigus situés entre les positions -17 et +6 par rapport au site d'initiation (position 1) de la transcription sont fortement conservés. Ces nucléotides sont même identiques chez les 5 promoteurs de classe III, qui sont les plus efficaces *in vivo* et *in vitro*. Parmi les 11 promoteurs différents, la plupart divergent par les nucléotides compris entre les positions +3 à +6. Parmi les nucléotides compris entre les positions -16 et -1, neuf sont complètement conservés, et quatre autres sont conservés dans 16 promoteurs. Ces constatations suggèrent que ces 13 nucléotides sont des facteurs importants de la définition d'un promoteur. Ces conclusions ont été confirmées par la mise en évidence de l'efficacité d'un promoteur après coupure de l'ADN en amont de la position -21 (Osterman, H. L. et al. (1981) *Biochemistry* **20**, 4884-4892) et -17 (Martin, C. T. et al. (1987) *Biochemistry* **26**, 2690-2696). Par contre, ces mêmes publications montrent qu'une coupure en amont de -10 ou -12 abolit cette efficacité.

L'ARN polymérase du phage T3 est presque aussi bien connue que la T7 ARN polymérase. Ces deux enzymes ont une structure très similaire (80 % d'identité) (McGraw, N. J. et al.(1985) *Nucleic Acids Res.* **13**, 6753-6766) mais possèdent pourtant une spécificité de matrice presque absolue. Les séquences de 11 promoteurs spécifiques pour la T3 ARN polymérase ont été déterminées et sont présentées dans le tableau 2 ci-dessous (brin non-matrice).

### Tableau 2

*Promoteurs pour la T3 ARN polymérase*

| Promoteur | Nucléotide | | |
|---|---|---|---|
| | -20 | -10 | 1 |
| | | . | . |
| Séquence conservée | TATTAACCCTCACTAAAGGGAGA | | |
| | -20 | -10 | 1 |
| | | . | . |
| | GTC | TATTTACCCTCACTAAAGGGAAT | AAGG |
| | TAG | CATTAACCCTCACTAACGGGAGA | CTAC |
| | TAC | AGTTAACCCTCACTAACGGGAGA | GTTA |
| | AAG | TAATAACCCTCACTAACAGGAGA | ATCC |
| | GGG | CATTAACCCTCACTAACAGGAGA | CACA |
| | GCC | TAATTACCCTCACTAAAGGGAAC | AACC |
| | TAC | AATTAACCCTCACTAAAGGGAAG | AGGG |
| | TCT | AATTAACCCTCACTAAAGGGAGA | GACC |
| | ACC | TAATTACCCTCACTAAAGGGAGA | CCTC |
| | GTG | AATTAACCCTCACTAAAGGGAGA | CACT |
| | TTG | CATTAACCCTCACTAAAGGGAGA | GAGG |

Actuellement, 4 séquences différentes de promoteurs pour l'ARN polymérase du phage SP6 ont été mises en évidence par Brown, J. E., et al. (*Nudeic Acids Res.* **14**, 3521-3526 (1986)). L'ARN polymérase du phage SP6 présente aussi beaucoup de similitudes avec l'ARN polymérase du phage T7. Ces séquences sont présentées au tableau 3 ci-dessous (brin non-matrice).

Tableau 3

*Promoteurs pour la SP6 ARN polymérase*

| Promoteur | Nucléotide | | |
|---|---|---|---|
| | | -10 | 1 |
| Séquence conservée | | ATTTAGGTGACACTATAGAAGGG | |
| | -20 | -10 | 1 |
| pSP64 | ACACATACG | ATTTAGGTGACACTATAGAATAC | AA |
| pJEB1 | TAATTGCCT | ATTTAGGTGACACTATAGAAGGG | AG |
| pJEB4 | GGACTTGGT | AATTAGGGGACACTATAGAAGGA | GG |
| pJEB6 | GTGTCTCTT | ATTTAGGGGACACTATAGAAGAG | AG |

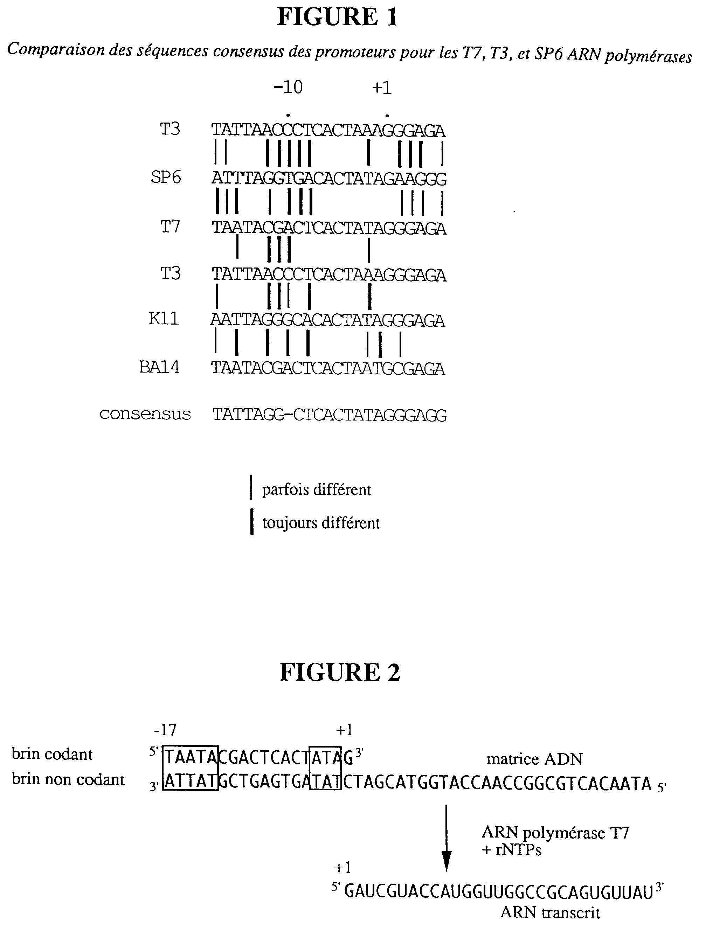

De même que pour la T7 ARN polymérase, les séquences des promoteurs pour la T3 ARN polymérase, et pour la SP6 ARN polymérase sont très similaires, notamment entre les positions -17 et +6. La comparaison des séquences de ces trois promoteurs (Figure 1) montre l'existence d'une séquence commune de la position -7 à -3 voir à ce propos notamment : BROWN, J.E. et al., *Nucleic Acid Res.*, 14, 3521-3526 (1986), et Bailey et al., Proc. Natl. Acad. Sci. USA 80 : 2814-2818 (1983).

Il est donc possible de considérer que les diverses ARN polymérases phagiques étudiées ci-dessus font partie d'une famille d'ARN polymérases qui reconnaissent des promoteurs présentant une séquence consensus de la position -17 à la position +6, et notamment de -17 à -1.

Pour obtenir l'ARN correspondant à une séquence d'ADN donnée sous l'action d'une ARN polymérase, il est nécessaire de mettre cette séquence sous la dépendance du promoteur de cette ARN polymérase. C'est l'étape dite d'installation d'un promoteur, juste en amont de la séquence à transcrire. Cette installation, dans l'état actuel de l'art, nécessite l'utilisation de méthodes laborieuses.

La méthode la plus classique d'installation d'un promoteur est le clonage de la séquence à transcrire dans un vecteur contenant un promoteur pour une ARN polymérase phagique en amont d'un site de clonage. Plusieurs vecteurs de ce type sont commercialisés, comme par exemple pT3/T7-LUC (Clontech Laboratories, Inc.), ou lambda ZAP II, Uni-ZAP XR, lambda DASH II, lambda FIX II, pWE 15 et superCos cosmid (Stratagene Cloning System), ou pT7-0, pT71, ou pT7-2 (United States Biochemical), ou encore pT7/T3a-18 ou pT7/T3a-19 (GIBCO BRL) ou décrits dans des publications, comme les vecteurs pET (Rosenberg, A.H. et al., Gene 56, 125-135 (1987)). C'est après obtention du fragment d'ADN à cloner, insertion dans le vecteur et amplification du vecteur que la transcription peut être effectuée. Cette méthode permet de transcrire n'importe quelle séquence. Cependant elle est très laborieuse, et ne permet pas d'obtenir n'importe quelle extrémité sur l'ARN, car la localisation de cette dernière est imposée par le site de restriction enzymatique utilisé pour le clonage.

Certaines méthodes connues pour la synthèse *in vitro* d'oligoribonucléotides nécessitent la synthèse par voie chimique de deux oligodésoxynucléotides complémentaires comprenant un promoteur pour une ARN polymérase phagique. Il a été démontré (Milligan et al., article cité), qu'une matrice partiellement simple brin, qui est double brin uniquement sur la séquence promoteur, de la position +1 à -14, est aussi active en transcription qu'une matrice double brin. Ainsi, la méthode nécessite la synthèse d'un nucléotide de 15 mer comprenant la séquence du brin non-matrice d'un promoteur phagique de la position -14 à +1, et d'un deuxième oligonucléotide contenant à son extrémité 3', la séquence complémentaire du premier, et sur sa région 5', la séquence complémentaire de l'oligoribonucléotide que l'on désire synthétiser. Comme la synthèse chimique ne permet pas d'obtenir des oligonucléotides de bonne qualité si leur taille est supérieure à 70 bases, cette technique n'est pas applicable pour les oligoribonucléotides de plus de 55 bases, si on utilise les séquences de promoteurs déjà décrites. De plus la méthode nécessite de connaître exactement la séquence de l'ARN que l'on veut synthétiser.

Une autre méthode d'installation d'un promoteur sur une séquence, par ligation, est possible (Leary, S. et al., Gene 106, 93-96, (1991)) si cette séquence possède une extrémité 3' bien définie. Il s'agit de l'hybridation sur cette extrémité 3' d'un oligonucléotide portant la séquence du brin non-matrice du promoteur, suivie par la séquence complémentaire de l'extrémité 3' de la séquence cible, sur une longueur suffisante pour permettre l'hybridation. Un deuxième oligonucléotide intervient, dont la séquence est complémentaire de la région promoteur du premier, et qui porte un groupement phosphate en 5'. L'hybridation des deux oligonucléotides et de la cible met en contact l'extrémité 5' phosphate de l'oligonucléotide portant la séquence du brin matrice du promoteur, et l'extrémité 3' OH de la cible. Sous l'action d'une ligase, la liaison phosphodiester entre ces deux extrémités est établie, permettant la formation d'un complexe dans lequel la cible se trouve sous le contrôle du promoteur.

L'apparition de la technique de "réaction d'amplification en chaîne par polymérase" (PCR), a permis la création de nouvelles méthodologies plus performantes pour l'installation d'un promoteur. La matrice de transcription est synthétisée par PCR. L'amorce en amont apporte le promoteur pour l'ARN polymérase phagique et définit l'extrémité 5' du produit de transcription (ou transcrit), alors que l'amorce en aval définit l'extrémité 3' de l'ADN amplifié et du transcrit. Cette technique permet d'obtenir un ARN de taille et d'extrémité 3' quelconques.

L'installation d'un promoteur phagique, grâce à une réaction inspirée du cycle des rétrovirus, est possible à partir d'un ARN (Kwoh, D. Y. et al., P.N.A.S. USA; 86, 1173-1177, (1989)). Une amorce amont portant la séquence du promoteur sert à synthétiser l'ADN complémentaire de l'ARN grâce à une transcriptase inverse. L'ARN du duplex ARN:ADN ainsi formé est digéré par la RNase H. L'ADN simple brin ainsi libéré s'hybride avec la deuxième amorce qui, grâce à la transcriptase inverse, permet la synthèse du deuxième brin d'ADN complémentaire. Cependant cette première étape ne permet pas de définir l'extrémité 3' de l'ARN. C'est la deuxième synthèse par les mêmes enzymes et par le même procédé, d'une deuxième génération de matrice pour l'ARN phagique, à partir des premiers transcrits obtenus, qui permet d'obtenir une extrémité 3' strictement définie par la seconde amorce utilisée.

L'un des buts de l'invention est de réduire au maximum les difficultés d'installation du promoteur, à l'aide de promoteurs modifiés de séquence courte, permettant de réduire les contraintes de séquence sur la séquence cible à transcrire. L'invention permet aussi d'obtenir des promoteurs de différentes efficacités, utilisables pour la transcription à des taux différents de séquences différentes, dans un même milieu réactionnel.

L'utilisation des promoteurs modifiés selon l'invention permet d'obtenir la transcription d'une séquence quelconque d'acide nucléique, désoxyribonucléique ou ribonucléique.

L'invention a donc pour objet un oligonucléotide utilisable comme brin non-matrice de promoteur, dans la transcription d'une séquence d'une cible nucléotidique, en présence d'une ARN polymérase phagique ayant un promoteur naturel spécifique, à partir d'un site de ladite cible qui n'est normalement pas un site d'initiation de la transcription pour ladite ARN polymérase phagique, laquelle est choisie parmi celles des phages dont les ARN polymérases ont des promoteurs spécifiques comprenant une séquence consensus au moins depuis la position -17 jusqu'à la position -1, caractérisé par le fait :

- qu'il comprend une séquence de coeur flanquée à l'une au moins de ses extrémités d'une séquence nucléotidique capable d'hybridation avec une séquence de la cible,
- que ladite séquence de coeur est constituée par une séquence de 6 à 9 nucléotides consécutifs choisis dans la région -12 à -4 du brin non-matrice dudit promoteur spécifique, ou une séquence suffisamment homologue permettant de conserver la fonctionnalité de ladite ARN polymérase,
- qu'une séquence flanquante est complémentaire d'une première région de la cible, et que l'autre séquence flanquante, lorsqu'elle est présente, est complémentaire d'une seconde région de la cible, lesdites première et seconde régions étant séparées, sur la cible, par une séquence ayant un nombre de nucléotides égal au nombre de nucléotides de la séquence de coeur,
- et que le nombre de nucléotides de la région flanquante, ou la somme du nombre de nucléotides des régions flanquantes, est au moins suffisamment élevé pour que ledit oligonucléotide puisse s'hybrider avec la cible à la température d'utilisation de l'ARN polymérase.

Les ARN polymérases phagiques présentant les caractéristiques indiqués ci-dessus sont du type comportant une seule sous-unité et ayant une masse moléculaire voisine de 100 kDa. L'invention s'étend bien entendu à l'utilisation d'ARN polymérases virales, non phagiques, présentant des caractéristiques analogues, et à la modification des promoteurs spécifiques desdites ARN polymérases virales.

On a en effet découvert qu'une séquence courte, telle que la séquence de coeur mentionnée ci-dessus, est capable de fonctionner comme promoteur de l'ARN polymérase. Cette découverte présente un caractère surprenant dans la mesure où la majeure partie de la séquence de coeur, telle que définie ci-dessus, correspond à des séquences très variables des promoteurs sauvages, notamment entre les positions -8 et -12, comme le montre l'examen de la figure 1.

La séquence de coeur étant relativement courte, cela augmente les chances de rencontrer de façon aléatoire sur une séquence cible, une séquence complémentaire de la séquence de coeur, ou suffisamment complémentaire de la séquence de coeur pour permettre l'hybridation au moins partielle avec celle-ci. Il en résulte une possibilité d'effectuer une transcription en un site qui n'est normalement pas un site de début de transcription, avec le promoteur sauvage, non modifié. Cette possibilité se trouve encore augmentée par la présence de la région flanquante ou des régions flanquantes, qui sont par construction complémentaires de la cible et qui contribue de façon importante à l'hybridation du promoteur modifié sur la cible.

De préférence, la séquence de coeur est choisie parmi des séquences de 6 à 9 nucléotides d'un promoteur naturel existant. Toutefois, des séquences homologues, différant par exemple d'un ou deux nucléotides par rapport au promoteur naturel, peuvent aussi être utilisées, notamment pour améliorer, le cas échéant, l'appariement de la séquence de coeur avec la séquence correspondante de la cible, dans la mesure où la séquence de coeur est suffisamment homologue de la séquence naturelle correspondante pour conserver la fonctionnalité de l'enzyme, c'est-à-dire dans la mesure où l'enzyme peut fonctionner avec un promoteur ayant une séquence de coeur ainsi modifiée, ce qui peut être facilement vérifié par de simples expériences de routine.

L'oligonucléotide de l'invention peut être constitué de bases naturelles, désoxyribonucléiques ou ribonucléiques, ou être constitué au moins en partie de bases modifiées. Il peut être constitué notamment d'oligonucléotides modifiés par couplage avec du psoralène, ou encore être constituées de PNA (peptide nucleic acids).

Lorsque deux séquences flanquantes sont présentes respectivement en amont et an aval de la séquence de coeur, elles sont complémentaires de deux régions de la cible espacées, sur la cible, d'un nombre de nucléotides égal au nombre de nucléotides de la séquence de coeur.

Grâce à la région flanquante, ou aux régions flanquantes, l'hybridation de l'oligonucléotide avec la cible permet généralement d'effectuer la transcription, y compris dans le cas où la séquence de coeur n'est pas complémentaire, même partiellement, de la région en regard sur la cible, dans le duplex cible-oligonucléotide. Cette possibilité est encore augmentée avec l'utilisation de bases modifiées pour la réalisation de la séquence de coeur et/ou des séquences flanquantes.

L'invention permet donc d'effectuer la transcription d'une séquence quelconque d'une cible donnée, en faisant débuter la transcription en un site qui ne serait normalement pas un site d'initiation de la transcription pour un promoteur non modifié.

Selon un mode de réalisation préféré, l'oligonucléotide de l'invention comporte une région flanquante amont dont le nombre de nucléotides est tel que la somme des nombres de nucléotides de la séquence de coeur et de la séquence flanquante amont est au moins égale à 10, et en particulier au moins égale à 11. Chaque région flanquante comporte de préférence au moins 5 nucléotides et en particulier de 5 à 12 nucléotides.

La séquence -12 à -4, dans laquelle peut être choisie la séquence de coeur, est en particulier l'une des séquences -12 à -4 du brin non matrice d'un promoteur sauvage. De telles séquences -12 à -4 sont par exemple les suivantes :

- GGTGACACT
- CGACTCACT
- ACCCTCACT
- GGGCACACT
- CAACTCACT
- CGACTCAGT
- GAACTCACT

Comme indiqué précédemment, la séquence de coeur peut être choisie à l'intérieur des séquences -12 à -4 de promoteurs sauvages. Il est aisément concevable que le rendement de la transcription pourra varier en fonction des séquences utilisées comme séquences de coeur. Il est toutefois possible, par sélection à l'aide de simples expériences de routine portant sur des essais de transcription, de construire un promoteur modifié permettant d'effectuer la transcription avec un rendement au moins égal à un seuil prédéterminé.

Bien entendu, les oligonucléotides dont la séquence de coeur correspondrait à une séquence d'un promoteur naturel, et dont les séquences amont et aval seraient fortuitement présentes dans ledit promoteur naturel, ne font pas partie de l'invention.

L'invention a également pour objet un procédé de préparation d'un oligonucléotide utilisable comme promoteur modifié pour ARN polymérase, caractérisé par le fait que l'on synthétise, selon les méthodes connues en soi, un oligonucléotide tel qu'il a été défini ci-dessus.

La présente invention permet de mettre une séquence quelconque sous le contrôle d'un tel promoteur modifié. Les méthodes utilisées pour cela sont notamment les méthodes déjà connues en soi, et en particulier les suivantes :

(1) le clonage de la séquence d'un tel promoteur en amont d'un site de clonage dans un vecteur d'expression plasmidique, permettant ensuite le clonage dans ce vecteur de la séquence que l'on désire transcrire sous le contrôle de ce promoteur.

(2) la synthèse d'un oligonucléotide comprenant la séquence du brin non matrice d'un promoteur modifié, et d'un deuxième oligonucléotide contenant à son extrémité 3' la séquence complémentaire du premier, et à son extrémité 5' la séquence complémentaire de l'oligoribonucléotide que l'on désire synthétiser. Comme la synthèse chimique est limitée en ce qui concerne la taille maximum des oligonucléotides, l'utilisation de promoteurs de séquence plus courte permet de synthétiser des oligoribonucléotides plus longs que si on utilise les séquences naturelles de promoteurs.

(3) la synthèse d'une matrice de transcription par Réaction de Polymérisation en Chaîne, l'amorce en amont apportant le promoteur modifié et définissant l'extrémité 5' du transcrit, l'amorce en aval définissant l'extrémité 3' de l'ADN amplifié et du transcrit.

(4) la synthèse d'une matrice de transcription par élongation, à l'aide d'une transcriptase inverse, d'une amorce amont portant la séquence du promoteur modifié hybridé sur un ARN, digestion par la RNAse H de l'ARN contenu dans le duplex ARN:ADN ainsi formé, et hybridation de l'ADN simple brin ainsi libéré avec la deuxième amorce qui, par élongation à l'aide de la transcriptase inverse, permet la synthèse du deuxième brin de la matrice et du promoteur modifié.

(5) l'installation par ligation d'un promoteur modifié sur une séquence acide désoxyribonucléique ou un acide ribonucléique si cette séquence possède une extrémité 3' bien définie, par hybridation sur cette extrémité 3' d'un oligonucléotide portant la séquence du brin non matrice du promoteur modifié, suivie par la séquence complémentaire de l'extrémité 3' de la séquence cible, sur une longueur suffisante pour l'hybridation, et hybridation sur ce premier oligonucléotide d'un deuxième oligonucléotide dont la séquence est complémentaire de la région promoteur modifié du premier, et qui porte un groupement phosphate en 5'. L'hybridation des deux oligonucléotides et de la cible met en contact l'extrémité 5' phosphate de l'oligonucléotide portant la séquence du brin matrice du promoteur et l'extrémité 3' OH de la cible. L'action d'une ligase permet alors la formation d'un complexe où la cible se trouve sous le contrôle du promoteur modifié.

La présente invention concerne également l'installation d'une séquence quelconque sous le contrôle d'un promoteur modifié, par une méthode, décrite ci-après, fondée sur l'utilisation de séquences double brin plus courtes que la séquence sauvage du promoteur, et/ou présentant des mésappariements amenés par des mutations sur le brin non codant, et/ou présentant des non appariements dus à une/des délétions sur le brin matrice du promoteur, et/ou portant d'autres modifications. En particulier, la présente invention concerne l'installation simplifiée d'un promoteur pour une ARN polymérase en amont d'un acide nucléique (appelée cible) de séquence donnée, de nature acide désoxyribonucléique ou acide ribonucléique, de façon à permettre la transcription de cette séquence par l'ARN polymérase concernée.

La méthode consiste à faire hybrider, sur la région 3' de la séquence à transcrire et sur la région immédiatement adjacente en 3' de cette séquence, un oligonucléotide de séquence partiellement ou totalement complémentaire de la cible, mais capable de s'hybrider avec la cible à la température d'utilisation de l'ARN polymérase.

L'oligonucléotide s'hybride au moins partiellement avec la cible grâce à l'hybridation sur la cible d'au moins la séquence flanquante (ou les séquences flanquantes).

La région "de coeur" peut donc présenter des non appariements ou des mésappariements avec la cible, c'est-à-dire des régions qui, de par leur séquence ou leur position ne sont pas en contact d'hybridation avec le brin matrice de la cible. En effet, l'hybridation de l'oligonucléotide, grâce à la séquence flanquante, ou aux séquences flanquantes, sur les régions de la cible qui leur sont complémentaires, permet la juxtaposition de la séquence "de coeur" avec une séquence éventuellement quelconque de la cible.

L'invention concerne également l'utilisation du promoteur modifié ci-dessus, comme brin non matrice de promoteur dans la transcription d'une séquence d'une cible nucléotidique capable de s'hybrider avec ledit oligonucléotide.

Pour la mise en oeuvre de cette utilisation, il convient de mettre en contact l'oligonucléotide avec la cible dans des conditions permettant la transcription, ce qui peut s'effectuer de différentes façons, et notamment comme suit :

(1) Si l'acide nucléique cible est un simple brin, l'hybridation peut être effectuée par mélange de la cible avec l'oligonucléotide, en solution dans un tampon qui est préférentiellement le tampon dans lequel s'effectuera la réaction de transcription, à une température permettant l'hybridation.

(2) Si l'acide nucléique cible est un double brin, l'hybridation peut être effectuée comme suit :

(2,1) Si l'oligonucléotide est un oligonucléotide non modifié, la cible devra être dénaturée, avant d'être hybridée à l'oligonucléotide dans les conditions décrites au paragraphe (1). La dénaturation peut être effectuée selon des procédés connus, en particulier par la chaleur (incubation durant 5 minutes à 95°C), ou encore par traitement alcalin (NaOH 0,1M durant 5 minutes), suivi de neutralisation avant hybridation avec l'oligonucléotide.

9

(2,2) Si l'oligonucléotide est un oligonucléotide modifié, et en particulier un PNA, l'hybridation ne nécessite pas de dénaturation préalable de la cible. Les séquences constituées en tout ou partie de PNA sont en effet capables de déplacer le brin complémentaire du brin sur lequel elles peuvent s'hybrider voir Nielsen, P.E. et al., Science 254, 1497-1500 (1991).

La transcription proprement dite est effectuée dans les conditions usuelles, qui sont bien connues, en présence de l'ARN polymérase appropriée et de ribonucléosides triphosphates, et qui ne seront pas rappelées ici.

La présente invention permet aussi d'effectuer une transcription différentielle de deux séquences différentes dans un même milieu réactionnel contenant une ARN polymérase reconnaissant le promoteur modifié. Parmi les promoteurs modifiés selon l'invention, certains permettent d'obtenir un taux de transcription égal à celui obtenu par utilisation d'un promoteur sauvage, et d'autres permettent au contraire d'obtenir un taux de transcription plus faible. La transcription différentielle a des utilisations multiples. En particulier elle permet :

(1) la transcription des brins codants et non codants d'une matrice qui porte à chaque extrémité ou sur une seule extrémité un promoteur modifié. L'introduction d'une différence dans l'efficacité des deux promoteurs permet de plus d'obtenir une synthèse en quantité plus importante d'un brin vis à vis de l'autre, si initialement, les deux brins ont la même capacité de transcription avec le même promoteur. Dans le cas où, au contraire, les deux brins n'ont pas la même efficacité de transcription avec un même promoteur, le choix de deux promoteurs à efficacité différente permet d'équilibrer le taux de transcription des deux transcrits.

(2) l'expression simultanée de protéines ou polypeptides que l'on veut obtenir dans certaines proportions au sein du milieu réactionnel, comme par exemple deux sous unités a et b d'une même holoenzyme $a_2b$. Pour cela, la séquence de la sous-unité a est mise sous le contrôle d'un promoteur sauvage, la séquence b est mise sous le contrôle d'un promoteur modifié dont l'efficacité est deux fois moindre par rapport à celle du promoteur de type sauvage.

Dans les dessins annexés :

La figure 1 est un tableau comparatif des séquences consensus des promoteurs pour les T3, T7 et SP6 ARN polymérases.

La figure 2 représente la séquence consensus (double brin) du promoteur de l'ARN polymérase T7 entre les positions -17 et +1. On a représenté le produit normal de la transcription. Le site de démarrage de transcription est indiqué par le symbole +1. Les zones encadrées définissent les sites sur lesquels ont été réalisées les délétions, sur le brin non-matrice (brin supérieur) et/ou sur le brin matrice (inférieur), conformément à l'exemple 1.

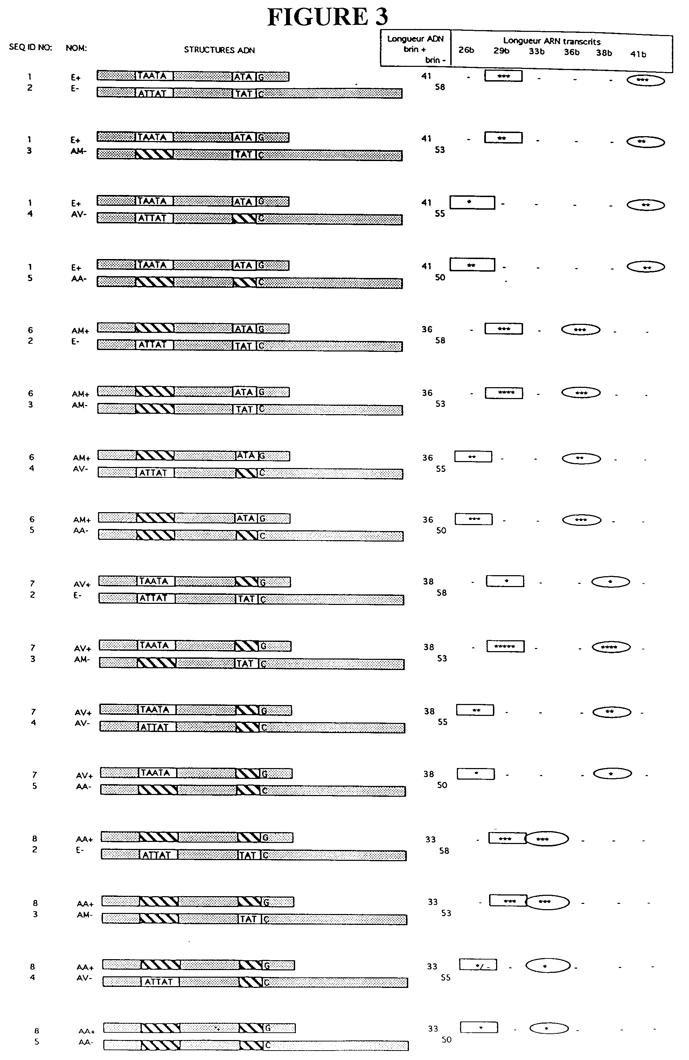

La figure 3 décrit l'ensemble des combinaisons de structures promotrices étudiées par transcription à l'aide de l'ARN polymérase T7. Les barres grisées schématisent les structures oligonucléotidiques utilisées. Pour chaque hybride, la barre supérieure correspond à une séquence décrite (de gauche à droite) de 5' vers 3' et la barre inférieure à une séquence décrite de 3' vers 5'. Les régions hachurées représentent les sites amputés sur l'un ou l'autre des brins, conformément à la figure 2. Le taux de production des produits de transcription relatifs à une taille donnée, visualisés par autoradiographie, est indiqué selon le mode suivant : (-), absence de produit de transcription ; (*) : taux très faible (**) : taux faible ; (***) : taux moyen ; (****) : taux élevé ; (*****) : taux très élevé. Les signaux encadrés par des rectangles correspondent aux produits de transcription de taille attendue par rapport au site d'initiation présumé et les signaux encerclés correspondent à un produit de transcription de taille identique au brin non-matrice (supérieur) des hybrides étudiés.

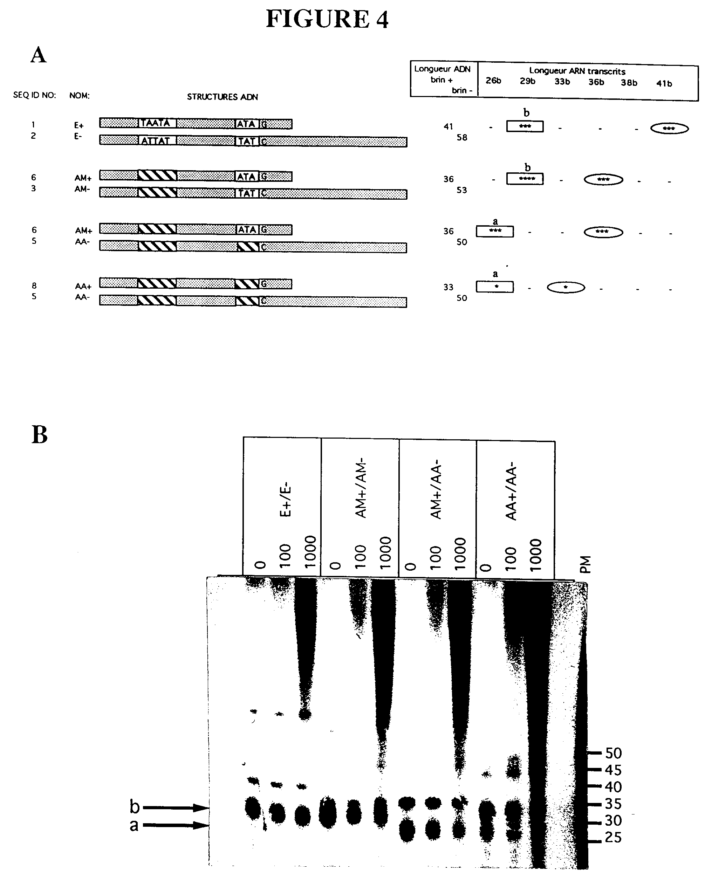

La partie A de la figure 4 décrit des combinaisons de séquences promotrices étudiées par transcription à l'aide d'ARN polymérase T7. Les barres grisées schématisent les structures oligonucléotidiques utilisées. Pour chaque hybride, la barre supérieure correspond à une séquence décrite (de gauche à droite) de 5' vers 3' et la barre inférieure à une séquence décrite de 3' vers 5'. Les régions hachurées représentent les sites amputés sur l'un ou l'autre des brins, conformément à la figure 2. Le taux d'obtention des produits de transcription relatifs à une taille donnée, visualisés par autoradiographie, est indiqué comme à la figure 3. La partie B de la figure 4 représente l'autoradiographie obtenue à l'issue des expériences schématisées à la figure 4A. Les signaux de transcription spécifiquement attendus sont indiqués par des flèches (a : 26 nucléotides; b : 29 nucléotides), conformément à la partie A. Les chiffres au dessus de chaque piste indiquent la quantité d'ADN exogène utilisée dans l'essai considéré. Les tailles des marqueurs de poids moléculaire (PM) sont indiquées sur la partie droite de l'autoradiogramme.

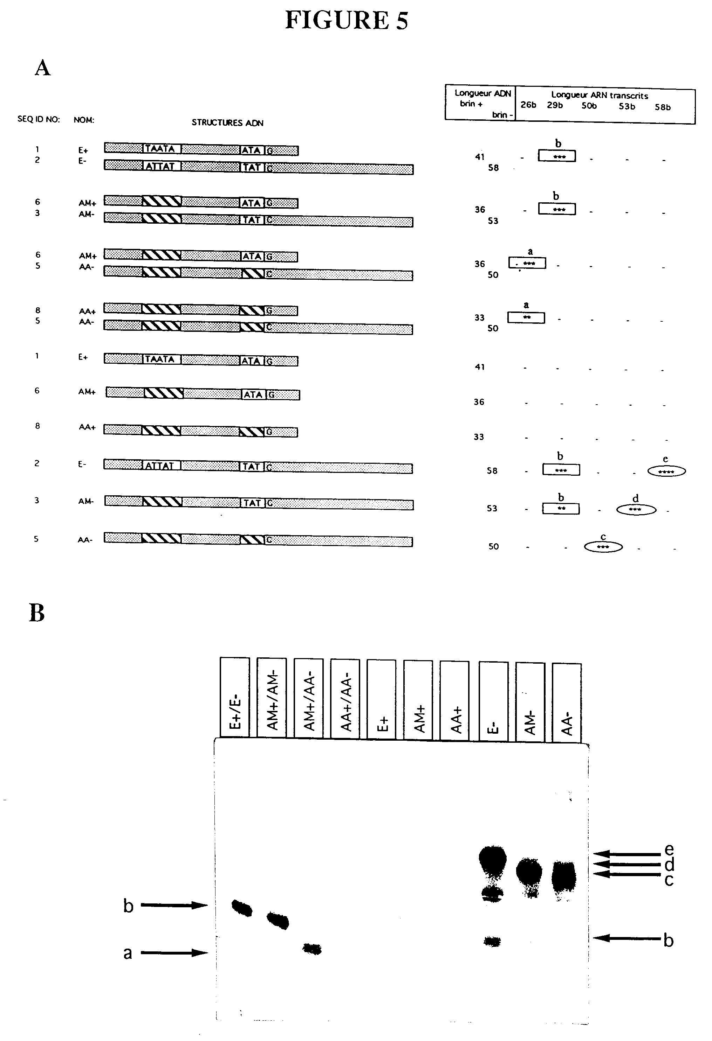

La partie A de la figure 5 décrit des combinaisons de séquences promotrices étudiées par transcription à l'aide de l'ARN polymérase T7, avec les mêmes conventions que pour la figure 4. La partie B représente l'autoradiographie obtenue à l'issue des expériences schématisées à la figure 5A. Les signaux de transcription spécifiquement attendus sont indiqués par des flèches (a : 26 nucléotides; b : 29 nucléotides; c : 50 nucléotides, d : 53 nucléotides, e : 58 nucléotides), conformément à la partie A.

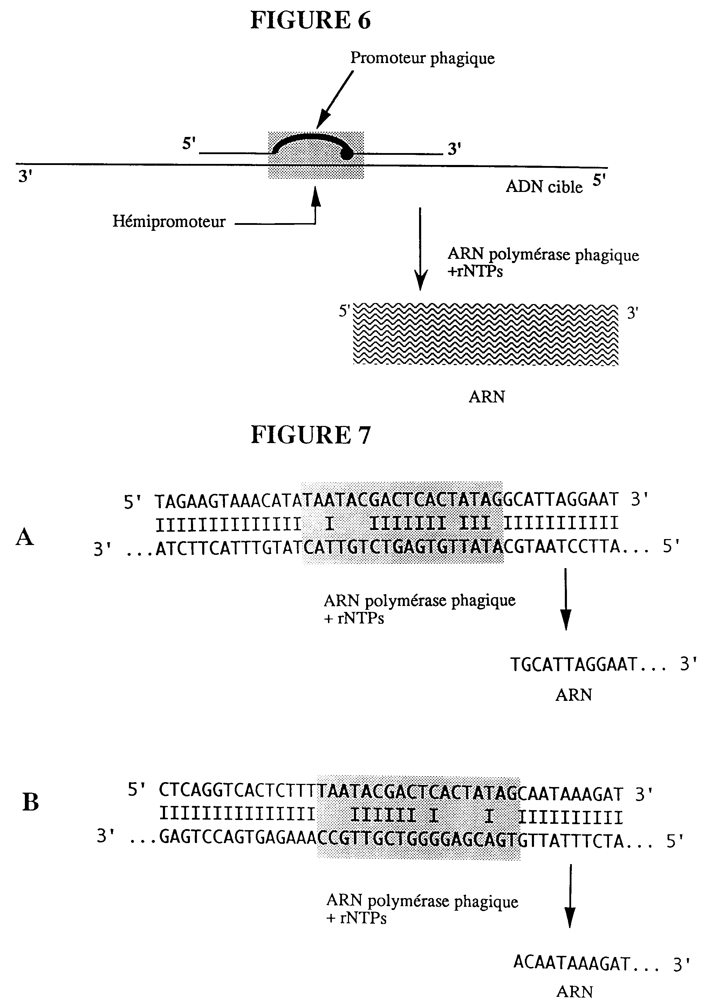

La figure 6 décrit schématiquement le fonctionnement d'un promoteur modifié selon l'invention, avec l'installation d'une séquence promotrice modifiée (brin non-matrice ; en trait gras) sur un site quelconque d'une séquence cible afin de définir un site d'initiation spécifique de la transcription. La séquence représentée en trait gras est maintenue sur la séquence cible par l'hybridation de séquences flanquantes complémentaires de la séquence cible. La région promotrice, sur laquelle l'ARN polymérase se fixe, est matérialisée par un rectangle grisé. Les ARN obtenus par transcription en présence de ribonucléotides triphosphates (rNTPs) sont schématisés par des lignes ondulées. Le site de démarrage de transcription est figuré par un rond noir.

La partie A de la figure 7 représente une structure promotrice modifiée créée par hybridation d'un oligonucléotide sur la séquence du génome de HIV (gène *pol*). Cet oligonucléotide est composé de deux parties flanquantes complémentaires de la cible et d'une séquence identique au consensus du promoteur de l'ARN polymérase du phage T7 (17 bases). Les identités de séquences sont matérialisées par des bâtonnets (I) et la partie en caractère gras sur fond grisé représente la structure partiellement double brin correspondant à une structure "hémipromotrice". La partie B de la figure 7 représente une structure promotrice modifiée crée par hybridation d'un oligonucléotide sur la séquence du génome de HIV (gène *gag*). Cet oligonucléotide est composé de deux parties flanquantes complémentaires de la cible et d'une séquence identique au consensus du promoteur de l'ARN polymérase du phage T7 (17 bases). Les identités de séquences sont matérialisées par des bâtonnets (I) et la partie en caractère gras sur fond grisé représente la structure partiellement double brin correspondant à une structure "hémipromotrice". Les ARN transcrits en présence d'ARN polymérase T7 et de ribonucléotides (rNTPs) sont indiqués. Des points de suspension symbolisent une extrémité d'un acide nucléique qui peut ne pas être d'une longueur définie.

Les exemples suivants illustrent l'invention.

## EXEMPLE 1 : ANALYSE DE LA FONCTIONNALITÉ DE PROMOTEURS MODIFIÉS

Différentes structures de promoteurs d'ARN polymérases phagiques (ADN - dépendantes), et plus particulièrement du phage T7 ont été étudiées. Ces structures sont obtenues par l'association de différentes séquences oligodésoxynucléotidiques synthétisées par voie chimique avec l'appareil Applied Biosystems 394 DNA/RNA Synthesizer. La liste de ces séquences (SEQ ID No: 1 à SEQ ID No:8) est indiquée en annexe.

La préparation des matrices ADN étudiées a été réalisée à partir de solutions contenant $2,5 \times 10^{14}$ copies d'oligodésoxynucléotides (ODN). Les différentes solutions nécessaires à la construction de la matrice sont associées dans un tube de microcentrifugeuse puis séchées sous vide. L'ensemble est repris dans 10 $\mu$l de tampon Tris-HCl 50 mM (pH 7.5) et remis en suspension. L'hybridation des structures possédant une complémentarité de séquence même partielle est réalisée par dénaturation à 95°C pendant 5 minutes, centrifugation (14000 x g, 10 secondes), puis descente à température ambiante pendant 10 minutes. A ce stade, les matrices peuvent être utilisées ou stockées à -20°C. Les essais de transcription sont réalisés à partir de 2 $\mu$l d'une dilution au 1/10$^{ème}$ des solutions précédentes, soit $5 \times 10^{12}$ copies de matrice simple ou double brin.

Les réactions de transcription sont réalisées dans le tampon suivant : Tris-HCl 40 mM (pH 8,1), spermidine 1 mM, dithiothreitol (DTT) 5 mM, Triton X-100 0,01 %, polyéthylèneglycol (PEG, poids moléculaire 4000) 80 mg/ml, albumine de sérum bovin (BSA) 50 $\mu$g (Boehringer). Le volume réactionnel final est de 20 $\mu$l et contient ATP, CTP, GTP, UTP (4 mM chaque), 5 $\mu$Ci de [$\alpha$-$^{32}$P]UTP, MgCl$_2$ 20 mM, 5 Unités d'ARN polymérase T7 (New England Biolabs) et 12 Unités de RNA Guard (Pharmacia). L'incubation est réalisée à 37°C pendant 2 heures.

Une fraction de 5 $\mu$l du mélange réactionnel est prélevée et mélangée avec 5 $\mu$l de la solution suivante : formamide 98 %, Xylène Cyanol 0,083 %, Bleu de Bromophénol 0,083 %, EDTA 10 mM pH 5,0. Les échantillons sont chauffés à 65°C pendant 5 minutes puis refroidis rapidement sur de la glace. Ils sont analysés par électrophorèse (2 heures, 150 Volts) en gel de polyacrylamide 12-15 %, urée 7 M. Les gels sont ensuite traités pendant 20 minutes dans une solution d'acide acétique/méthanol (10/20, %/%), séchés sous vide et autoradiographiés pendant 12 heures à -80°C.

Les différentes combinaisons étudiées permettent de déterminer l'influence de délétions situées dans les régions amont (AM) et aval (AV) de la séquence consensus du promoteur de l'ARN polymérase du phage T7, sur l'efficacité de transcription d'une matrice ADN définie. Les combinaisons permettent également de déterminer l'influence respective de chacune de ces délétions selon qu'elles sont situées sur le brin non-matrice ou matrice du promoteur double brin (figure 2). Les diverses associations étudiées dans cet exemple sont schématiquement représentées à la figure 3.

L'analyse comparative des résultats issus des combinaisons E$^+$/E$^-$, E$^+$/AM$^-$, AM$^+$/E$^-$ et AM$^+$/AM$^-$ (figure 3) permet de déterminer l'influence des délétions en amont du coeur (-4 à -12) du promoteur. Elle montre dans ce cas qu'une délétion sur le brin non-matrice a, seule, peu d'effet mais qu'une délétion sur le brin matrice, seule, réduit l'activité du promoteur. Cependant, une double délétion (brin non-matrice et matrice) rétablit un niveau de transcription normal, ce qui montre que la partie amont du promoteur peut être substituée par une séquence double brin quelconque sans modification de l'activité de ce dernier. Le promoteur de l'ARN polymérase du phage T7 peut donc être modifié en amont de la position -12 sans incidence sur le rendement de transcription, ni modification du site d'initiation de celle-ci.

L'analyse comparative des résultats issus des combinaisons E$^+$/E$^-$, E$^+$/AV$^-$, AV$^+$/E$^-$ et AV$^+$/AV$^-$ (figure 3) permet de déterminer l'influence des délétions en aval du coeur (positions -4 à 12) du promoteur. La délétion du promoteur en aval induit un décalage du site d'initiation de transcription par rapport au site du promoteur sauvage (C, correspondant à la position +1). En effet, un transcrit de plus petite taille (26 au lieu de 29 nucléotides) est observé dans tous les cas où un promoteur comprend une délétion aval sur le brin matrice. Cela démontre que le positionnement de l'ARN polymérase T7 sur le coeur du promoteur impose la localisation du site de démarrage de transcription sur le brin matrice. La partie aval du promoteur (-1 à -3) peut donc être enlevée et remplacée par une séquence différente de celle définie dans le consensus global de 17 paires de bases, ce qui démontre qu'un promoteur ainsi modifié conserve sa fonctionnalité, même si le rendement de la réaction est légèrement moindre.

L'analyse comparative des résultats issus des combinaisons E$^+$/E$^-$, E$^+$/AM$^-$, E$^+$/AV$^-$ et E$^+$/AA$^-$ (figure 3) permet de déterminer l'influence des délétions par rapport à leur localisation respective sur le brin matrice du promoteur. Une délétion située en amont ne modifie pas la taille du transcrit, mais atténue légèrement l'efficacité de transcription par rapport au promoteur sauvage. En revanche, une délétion située en aval conduit à l'obtention d'un produit de transcription de taille plus courte (déplacement du site de début de transcription) et affecte l'efficacité de transcription de plus de 50 %. La double délétion (amont et aval) produit un transcrit plus court mais son efficacité de transcription est similaire à celle correspondant à la délétion situé en aval. En d'autres termes, il n'y a pas d'effet cumulatif des deux types de délétion.

L'analyse comparative des résultats issus des combinaisons E$^+$/E$^-$, AM$^+$/E$^-$, AV$^+$/E$^-$ et AA$^+$/E$^-$ (figure 3) permet de déterminer l'influence des délétions sur le brin non-matrice du promoteur. Une délétion située en amont ne modifie pas l'efficacité de transcription par rapport au promoteur sauvage. En revanche, une délétion située en aval réduit de plus de 50 % l'efficacité de transcription. L'effet des deux mutations n'est pas cumulatif puisqu'une double délétion (amont et aval) permet d'obtenir une transcription presque aussi forte que le témoin.

L'analyse comparative des résultats issus des combinaisons E$^+$/E$^-$, AM$^+$/AV$^-$, AM$^+$/AA$^-$, AA$^+$/AV$^-$, AV$^+$/AM$^-$, AA$^+$/AM$^-$, AV$^+$/AA$^-$, AM$^+$/AM$^-$, AV$^+$/AV$^-$ et AA$^+$/AA$^-$ (figure 3) permet d'étudier la fonctionnalité de structures promotrices possédant au moins une délétion sur chaque brin (non-matrice et matrice). Elle permet de constater qu'un promoteur homoduplex dépourvu de sa partie amont (sur les deux brins), ainsi qu'un promoteur dépourvu de sa partie aval (sur les deux brins) conservent une bonne fonctionnalité par rapport au promoteur sauvage. De même, tout promoteur dépourvu de sa partie aval sur le brin matrice (mais non dépourvu sur le brin non-matrice) est fonctionnel. Cela démontre qu'il est possible de définir un promoteur modifié d'ARN polymérase phagique à partir d'une séquence minimum présente sur un ADN cible. Enfin, même si l'efficacité d'un promoteur homoduplex doublement amputé (en amont et en aval sur les deux brins) est réduite par rapport au promoteur sauvage, il faut constater que ce type de structure minimum permet néanmoins d'obtenir des transcrits avec une efficacité non négligeable.

Dans des conditions analogues, il est possible de montrer que le brin matrice du promoteur permet, sous forme de simple brin, d'obtenir un transcrit de taille identique à celui obtenu à l'aide d'un promoteur double brin. Une séquence simple brin matrice d'un promoteur possède donc l'information nécessaire à la fixation de l'ARN polymérase du phage T7 et à l'initiation de la transcription.

En résumé, les promoteurs bicaténaires raccourcis homo- ou hétéro-duplex conservent une grande fonctionnalité par rapport au promoteur sauvage. En particulier, une délétion sur les deux brins en amont n'a pas d'influence sur le rendement de transcription. Une délétion en aval sur le brin matrice affecte peu le rendement de transcription mais modifie simplement la taille du transcrit par décalage du site d'initiation de transcription sur la matrice. La conservation de la partie aval sur le brin non-matrice seul du promoteur (localisation -3 à -1), au niveau de la séquence $^{5'}$TATA$^{3'}$, permet de conserver une efficacité de transcription proche de celle obtenue à l'aide du promoteur sauvage. Néanmoins, il apparaît clairement d'après ces données qu'il est possible d'obtenir des promoteurs modifiés de plus petite taille permettant d'initier la transcription à partir d'un site donné.

## EXEMPLE 2 ANALYSE QUALITATIVE DE LA SPÉCIFICITÉ DE TRANSCRIPTION A PARTIR DE PROMOTEURS MODIFIÉS

Conformément à l'exemple 1, la spécificité de transcription *in vitro* de promoteurs modifiés a été étudiée en présence d'ADN exogène de sperme de saumon à différentes concentrations par essai. Les associations E$^+$/E$^-$, AM$^+$/AM$^-$, AM$^+$/AA$^-$ et AA$^+$/AA$^-$ ont été étudiées en présence de 0, 10, 100 et 1000 ng d'ADN exogène (figure 4). Cette étude permet de démontrer, sur quelques cas particuliers choisis dans l'exemple 1, que la transcription effectuée *in vitro* est intrinsèquement liée à la présence d'une structure promotrice fonctionnelle et non à la présence d'une séquence d'ADN aléatoire disponible pour l'ARN polymérase T7. Les essais expérimentaux ont été réalisés de manière identique à ceux de l'exemple 1.

La présence d'ADN exogène en forte concentration (1000 ng/essai) conduit à un fort bruit de fond correspondant à des transcrits aspécifiques de grande taille (figure 4B). Néanmoins, l'efficacité de transcription de la cible spécifique n'est pas modifiée quantitativement, en présence ou en absence d'ADN exogène. Cela démontre que les structures promotrices étudiées permettent une fixation préférentielle et efficace de l'ARN polymérase T7, conduisant à la transcription spécifique d'une cible pourvue d'un promoteur. Cette observation s'accorde avec le fait que l'intensité du bruit de fond aspécifique est inversement proportionnelle à l'efficacité du promoteur en présence, c'est-à-dire l'intensité relative du produit de taille attendue. Ces éléments démontrent donc la fonctionnalité des promoteurs modifiés qui permettent une transcription spécifique d'une cible située en aval.

## EXEMPLE 3 ANALYSE QUALITATIVE DES ARNs TRANSCRITS À PARTIR D'UNE SÉQUENCE CIBLE CONTRÔLÉE PAR UN PROMOTEUR MODIFIÉ

L'analyse qualitative de l'efficacité relative de différents types de structures promotrices a été complétée par une détection spécifique des ARNs transcrits correspondant à la séquence située en aval de la structure promotrice. Pour ce faire, des essais transcriptionnels ont été réalisés conformément à l'exemple 1, à l'exception de toute incorporation de nucléotides modifiés. Les associations $E^+/E^-$, $AM^+/AM^-$, $AM^+/AA^-$, $AA^+/AA^-$, ainsi que les séquences simple brin $E^+$, $AM^+$, $AA^+$, $E^-$, $AM^-$, et $AA^-$ ont été étudiées. Suite à la réaction de transcription proprement dite en absence d'isotope, les échantillons sont chargés sur un gel d'électrophorèse. Après migration selon les conditions de l'exemple 1, les acides nucléiques sont transférés électriquement sur membrane de nylon (Hybond N, Amersham) à l'aide d'un appareil (Biorad) dans un tampon TBE x 0,5 pendant deux heures à 80 Volts. Les membranes sont séchées pendant 10 minutes à 80 °C puis les acides nucléiques sont fixés sur la membrane par irradiation UV à l'aide d'un appareil Stratalinker (Stratagene). Les membranes sont préhybridées à 42°C dans une solution de préhybridation. L'hybridation est réalisée avec une sonde oligonucléotidique (SEQ ID NO. 9) marquée radioactivement à son extrémité 3' par du $[\alpha\text{-}^{32}P]$ddATP en présence de terminale transférase et purifiée. L'hybridation est réalisée pendant 12 heures dans des conditions discriminantes ("stringentes") ne permettant pas d'hybridations aspécifiques et les lavages sont également réalisés de manière stringente. La membrane est ensuite envelop-pée dans un film de cellophane et autoradiographiée pendant 24 heures à -80°C. Les résultats obtenus sont présentés dans la figure 5. Ils indiquent clairement qu'un promoteur double brin raccourci en amont ($AM^+/AM^-$) est aussi efficace qu'un promoteur sauvage ($E^+/E^-$). De même, un promoteur raccourci en amont sur le brin non-matrice et en amont et aval sur le brin matrice ($AM^+/AA^-$) permet d'obtenir un transcrit spécifique, c'est-à-dire complémentaire de la sonde de détection de l'ARN cible. Le rendement d'obtention de ce transcrit est similaire à celui obtenu a l'aide du promoteur sauvage, ce qui confirme l'efficacité de ce promoteur modifié pour la transcription d'un ARN complémentaire de la matrice située en aval de ce celui-ci. L'ARN produit dans ces conditions possède une taille réduite de 3 nucléotides par rapport à l'ARN issu du promoteur sauvage. Ceci provient du décalage du site de démarrage de transcription sur le brin matrice, dû à la délétion aval sur ce dernier. Ce décalage ampute vraisemblablement des trois premières bases l'ARN transcrit, mais ne modifie pas la séquence située en aval puisqu'un signal d'hybridation de même intensité que dans le cas du promoteur sauvage est obtenu avec la sonde spécifique du transcrit attendu. Un transcrit de taille réduite de 3 nucléotides est également obtenu à partir du promoteur double brin amputé en amont et en aval ($AA^+/AA^-$). Comme attendu, les brins correspondant au brin non matrice du promoteur ($E^+$, $AM^+$, $AA^+$) ne permettent pas d'obtenir de transcrits spécifiques. En revanche, avec le brin matrice seul, sauvage ou amputé en amont ($E^-$ et $AM^-$, respectivement), on obtient un transcrit spécifique de taille attendue (29 nucléotides). Cela démontre pour la première fois la capacité de brins matrices des promoteurs phagiques à initier une transcription à un site précis. Un brin matrice de promoteur doublement amputé ($AA^-$) ne permet pas d'initier la transcription, ce qui montre l'importance de la séquence $^{5'}TAT^{3'}$ située dans la partie aval du consensus du promoteur T7. La capacité de transcription peut être rétablie par hybridation en trans d'un brin non-matrice du promoteur comportant au moins cette séquence aval du promoteur. Il est important de noter que ces promoteurs simple brin (brin matrice) conduisent tous à des initiations de transcription à d'autres sites que ceux défini par le positionnement de l'ARN polymérase sur son promoteur sauvage. Notamment, des transcrits comportant la séquence de l'ARN attendu, mais de longueur égale à celle du brin matrice, sont obtenus. Cela résulte de l'initiation d'une transcription à partir de l'extrémité 3' libre d'un oligonucléotide, comme précédemment démontré ; voir Schenborn, E.T. et Mierendorf, R.C. Jr., Nucleic Acid Research, 13, 6223-6236 (1985).

En conclusion, il s'avère que des promoteurs phagiques amputés en amont et en aval de leur consensus, simple brin ou double brin, sont capables d'induire spécifiquement la transcription à un site particulier.

Ainsi, conformément à l'invention, et comme le montre la partie expérimentale ci-dessus, il est possible de créer des structures de promoteurs phagiques modifiés ou partiellement conservés. Ces structures peuvent être réalisées notamment par hybridation d'un oligonucléotide composé successivement (de 5' vers 3') d'une région complémentaire de la cible simple brin (région flanquante), d'une séquence du brin non-matrice promoteur de l'ARN polymérase phagique, et éventuellement d'une seconde région flanquante complémentaire de la cible (figure 6). Un tel oligonucléotide peut jouer le rôle d'hémipromoteur (brin non-matrice de promoteur) pour la transcription à l'aide d'ARN polymérase. Selon la nature de la séquence cible en regard de à la séquence du promoteur (dans le duplex cible-promoteur), et afin de limiter l'effet des mésappariements de cette région sur la capacité de fixation de l'ARN polymérase, des bases modifiées (inosine, par exemple) peuvent être introduites dans des positions particulières de la séquence du promoteur. Ces modifications doivent être adaptées à chaque cas particulier de séquence cible afin de favoriser la fixation de l'ARN polymérase sur la structure "hémipromoteur". La notion d'hémipromoteur recouvre l'idée d'une reconnaissance d'une séquence promotrice imparfaitement appariée. L'ARN polymérase se fixe sur la structure hémipromoteur et réalise la transcription d'ARNs dont l'extrémité 5' (généralement un G) correspond à la première base situé en aval de la structure hémipromoteur (figure 6). Tout mode d'installation de ce type de structure "hémipromotrice", ainsi que l'utilisation d'une telle structure, font partie de l'invention.

L'étape éventuelle de dénaturation initiale d'un acide nucléique double brin à transcrire peut être effectuée de manière thermique, chimique ou enzymatique.

Il est aisé de trouver, au sein d'une séquence cible, des régions ayant une homologie suffisante avec la séquence consensus d'un promoteur d'ARN polymérase phagique, et à plus forte raison avec une séquence raccourcie, suffisante pour initier la transcription, comme montré ci-dessus, afin de créer une structure "hémipromotrice" par hybridation sur un brin cible d'une séquence promotrice de coeur flanquée d'au moins une séquence spécifique de la cible. Le génome du virus d'immunodéficience humaine (HIV) présente par exemple deux régions d'homologie significative avec la séquence consensus du promoteur de l'ARN polymérase du phage T7 ($^{5'}$TAATACGACTCACTATAG$^{3'}$). Ces séquences sont $^{5'}$GGCAACGACCCCTCGTCA$^{3'}$ et $^{5'}$TAATACGACTCACAATAT$^{3'}$, et se situent respectivement à l'intérieur des gènes *gag* et *pol*. Les bases en caractère gras sont identiques dans la séquence consensus du promoteur et dans les séquences déterminées par recherche d'alignements optimaux entre ledit consensus et la séquence totale du génome du HIV. L'hybridation d'une séquence contenant une partie de la séquence consensus (brin non-matrice) du promoteur de l'ARN polymérase du phage T7, flanquée de séquences complémentaires de la cible HIV, permet d'installer une structure "hémipromotrice" sur le génome du HIV et de réaliser, en présence d'ARN polymérase T7 et de ribonucléosides triphosphates (rNTPs) une transcription spécifique à partie du site ainsi défini (figure 7). Cet exemple illustre par conséquent la possibilité d'application de cette méthode d'initiation de transcription, à partir d'une séquence d'acide nucléique d'intérêt, potentiellement à tous les systèmes biologiques existants.

**LISTE DES SEQUENCES**

SEQ ID N° : 1 :

GATCGTACCA TGTAATACGA CTCACTATAG ATCGTACCAT G          41


SEQ ID N° : 2 :

ATAACACTGC GGCCAACCAT GGTACGATCT ATAGTGAGTC          40

GTATTACATG GTACGATC          58


SEQ ID N° : 3 :

ATAACACTGC GGCCAACCAT GGTACGATCT ATAGTGAGTC          40

GCATGGTACG ATC          53


SEQ ID N° : 4 :

ATAACACTGC GGCCAACCAT GGTACGATCA GTGAGTCGTA          40

TTACATGGTA CGATC          55


SEQ ID N° : 5 :

ATAACACTGC GGCCAACCAT GGTACGATCA GTGAGTCGCA          40

TGGTACGATC          50


SEQ ID N° : 6 :

GATCGTACCA TGCGACTCAC TATAGATCGT ACCATG          36


SEQ ID N° : 7 :

GATCGTACCA TGTAATACGA CTCACTGATC GTACCATG          38


SEQ ID N° : 8 :

GATCGTACCA TGCGACTCAC TGATCGTACC ATG          33


SEQ ID N° : 9 :

ATAACACTGCGGCCAAC          17


**Revendications**

1. Oligonucléotide utilisable comme brin non-matrice de promoteur, dans la transcription d'une séquence d'une cible nucléotidique, en présence d'une ARN polymérase phagique ayant un promoteur naturel spécifique, à partir d'un site de ladite cible qui n'est normalement pas un site d'initiation de la transcription pour ladite ARN polymérase phagique, laquelle est choisie parmi celles des phages dont les ARN polymérases ont des promoteurs spécifiques comprenant une séquence consensus au moins depuis la position -17 jusqu'à la position -1, caractérisé par le fait :

- qu'il comprend une séquence de coeur flanquée à l'une au moins de ses extrémités d'une séquence nucléotidique capable d'hybridation avec une séquence de la cible,
- que ladite séquence de coeur est constituée par une séquence de 6 à 9 nucléotides consécutifs choisis dans la région -12 à -4 du brin non-matrice dudit promoteur spécifique, ou une séquence suffisamment homologue permettant de conserver la fonctionnalité de ladite ARN polymérase,
- qu'une séquence flanquante est complémentaire d'une première région de la cible, et que l'autre séquence flanquante, lorsqu'elle est présente, est complémentaire d'une seconde région de la cible, lesdites première et seconde régions étant séparées, sur la cible, par une séquence ayant un nombre de nucléotides égal au nombre de nucléotides de la séquence de coeur,
- et que le nombre de nucléotides de la région flanquante, ou la somme du nombre de nucléotides des régions flanquantes, est au moins suffisamment élevé pour que ledit oligonucléotide puisse s'hybrider avec la cible à la température d'utilisation de l'ARN polymérase.

2. Oligonucléotide selon la revendication 1, caractérisé par le fait qu'il comporte une région flanquante amont dont le nombre de nucléotides est tel que la somme des nombres de nucléotides de la séquence de coeur et de la séquence flanquante amont est au moins égale à 10, et en particulier au moins égale à 11.

3. Oligonucléotide selon la revendication 1 ou 2, caractérisé par le fait que les régions flanquantes comportent chacune de 5 à 12 nucléotides.

4. Oligonucléotide selon l'une quelconque des revendications précédentes, caractérisé par le fait que la séquence -12 à -4 dudit brin non matrice dudit promoteur est choisie parmi l'une des séquences suivantes :
    - ACCCTCACT
    - GGTGACACT
    - CGACTCACT
    - GGGCACACT
    - CAACTCACT
    - CGACTCAGT
    - GAACTCACT

5. Procédé de préparation d'oligonucléotides utilisables comme brins non-matrice dans la transcription d'une séquence d'une cible nucléotidique, à partir d'un site qui n'est normalement pas un site d'initiation de la transcription pour ladite ARN polymérase phagique, caractérisé par le fait que l'on synthétise des oligonucléotides tels que définis dans l'une quelconque des revendications précédentes, que l'on effectue de façon connue des essais de transcription de ladite cible en utilisant comme brin non-matrice les oligonucléotides obtenus, et que l'on élimine les oligonucléotides qui ne permettent pas d'effectuer ladite transcription avec un rendement au moins égal à un seuil prédéterminé.

6. Utilisation d'un oligonucléotide tel que défini dans l'une quelconque des revendications 1 à 4 comme brin non matrice dans la transcription, en présence d'une ARN polymérase, à partir d'un site qui n'est normalement pas un site de début de transcription pour ladite ARN polymérase, d'une séquence d'une cible nucléotidique capable de s'hybrider avec ledit oligonucléotide.

# FIGURE 1

*Comparaison des séquences consensus des promoteurs pour les T7, T3, et SP6 ARN polymérases*

```
              -10           +1
              .            .
    T3     TATTAACCCTCACTAAAGGGAGA
           ||   ||||| |  | |||  |
    SP6    ATTTAGGTGACACTATAGAAGGG
            |||  | |||       |||  |
    T7     TAATACGACTCACTATAGGGAGA
            |   |||       |
    T3     TATTAACCCTCACTAAAGGGAGA
            |   ||| |     |
    K11    AATTAGGGCACACTATAGGGAGA
            | |  | |      || |
    BA14   TAATACGACTCACTAATGCGAGA

  consensus   TATTAGG-CTCACTATAGGGAGG
```

| parfois différent

| toujours différent

# FIGURE 2

```
           -17                  +1
brin codant      5' TAATACGACTCACTATAG 3'          matrice ADN
brin non codant  3' ATTATGCTGAGTGATATCTAGCATGGTACCAACCGGCGTCACAATA 5'
```

ARN polymérase T7
+ rNTPs

```
           +1
      5' GAUCGUACCAUGGUUGGCCGCAGUGUUAU 3'
                    ARN transcrit
```

# FIGURE 3

| SEQ ID NO: | NOM: | STRUCTURES ADN | Longueur ADN brin + brin - | Longueur ARN transcrits | | | | | |
|---|---|---|---|---|---|---|---|---|---|
| | | | | 26b | 29b | 33b | 36b | 38b | 41b |
| 1 | E+ | TAATA ... ATA G | 41 | - | *** | - | - | - | *** |
| 2 | E- | ATTAT ... TAT C | 58 | | | | | | |
| 1 | E+ | TAATA ... ATA G | 41 | - | ** | - | - | - | ** |
| 3 | AM- | ... TAT C | 53 | | | | | | |
| 1 | E+ | TAATA ... ATA G | 41 | * | - | - | - | - | ** |
| 4 | AV- | ATTAT ... C | 55 | | | | | | |
| 1 | E+ | TAATA ... ATA G | 41 | ** | - | - | - | - | ** |
| 5 | AA- | ... C | 50 | | | | | | |
| 6 | AM+ | ... ATA G | 36 | - | *** | - | *** | - | - |
| 2 | E- | ATTAT ... TAT C | 58 | | | | | | |
| 6 | AM+ | ... ATA G | 36 | - | **** | - | *** | - | - |
| 3 | AM- | ... TAT C | 53 | | | | | | |
| 6 | AM+ | ... ATA G | 36 | ** | - | - | ** | - | - |
| 4 | AV- | ATTAT ... C | 55 | | | | | | |
| 6 | AM+ | ... ATA G | 36 | *** | - | - | *** | - | - |
| 5 | AA- | ... C | 50 | | | | | | |
| 7 | AV+ | TAATA ... G | 38 | - | * | - | - | * | - |
| 2 | E- | ATTAT ... TAT C | 58 | | | | | | |
| 7 | AV+ | TAATA ... G | 38 | - | ***** | - | - | **** | - |
| 3 | AM- | ... TAT C | 53 | | | | | | |
| 7 | AV+ | TAATA ... G | 38 | ** | - | - | - | ** | - |
| 4 | AV- | ATTAT ... C | 55 | | | | | | |
| 7 | AV+ | TAATA ... G | 38 | * | - | - | - | * | - |
| 5 | AA- | ... C | 50 | | | | | | |
| 8 | AA+ | ... G | 33 | - | *** | *** | - | - | - |
| 2 | E- | ATTAT ... TAT C | 58 | | | | | | |
| 8 | AA+ | ... G | 33 | - | *** | *** | - | - | - |
| 3 | AM- | ... TAT C | 53 | | | | | | |
| 8 | AA+ | ... G | 33 | */- | - | * | - | - | - |
| 4 | AV- | ATTAT ... C | 55 | | | | | | |
| 8 | AA+ | ... G | 33 | * | - | * | - | - | - |
| 5 | AA- | ... C | 50 | | | | | | |

18

# FIGURE 4

**A**

| Longueur ADN brin + | | Longueur ARN transcrits | | | | | |
|---|---|---|---|---|---|---|---|
| brin - | 26b | 29b | 33b | 36b | 38b | 41b |

| SEQ ID NO: | NOM: | STRUCTURES ADN |
|---|---|---|

**B**

# FIGURE 5

**A**

**B**

# FIGURE 6

Promoteur phagique

Hémipromoteur

ADN cible

ARN polymérase phagique
+rNTPs

ARN

# FIGURE 7

A

```
5' TAGAAGTAAACATATAATACGACTCACTATAGGCATTAGGAAT 3'
   IIIIIIIIIIIIII I   IIIIII III IIIIIIIIIII
3' ...ATCTTCATTTGTATCATTGTCTGAGTGTTATACGTAATCCTTA... 5'
```

ARN polymérase phagique
+ rNTPs

TGCATTAGGAAT... 3'

ARN

B

```
5' CTCAGGTCACTCTTTTAATACGACTCACTATAGCAATAAAGAT 3'
   IIIIIIIIIIIIII   IIIIII I    I IIIIIIIIII
3' ...GAGTCCAGTGAGAAACCGTTGCTGGGGAGCAGTGTTATTTCTA... 5'
```

ARN polymérase phagique
+ rNTPs

ACAATAAAGAT... 3'

ARN

**Office européen des brevets**

# RAPPORT DE RECHERCHE EUROPEENNE

Numero de la demande

EP 94 40 2996

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,Y | BIOCHEMISTRY, vol.26, no.10, 19 Mai 1987, AM. CHEM. SOC.,WASHINGTON,DC,US; pages 2690 - 2696 C.T. MARTIN AND J.E. COLEMAN 'Kinetic analysis of T7 RNA polymerase-promoter interactions with small synthetic promoters' * page 2693, colonne de droite, ligne 28 - page 2694, colonne de gauche, ligne 27 * | 1-6 | C12N15/10 C12N15/11 C12Q1/68 C12Q1/70 |
| Y | EP-A-0 439 330 (IMPERIAL CHEMICAL INDUSTRIES PLC) 31 Juillet 1991 * page 5, ligne 37 - ligne 45; figures 2,3 * | 1-6 | |
| D,A | NUCL. ACIDS RES., vol.15, no.21, 11 Novembre 1987, IRL PRESS, OXFORD, ENGLAND; pages 8783 - 8798 J.F. MILLIGAN ET AL. 'Oligoribonucleotide synthesis using T7 RNA polymerase and synthetic DNA templates' * en entier * | 1-6 | |
| D,A | NUCL. ACIDS RES., vol.14, no.8, 25 Avril 1986, IRL PRESS, OXFORD, ENGLAND; pages 3521 - 3526 J.E. BROWN ET AL. 'Sequence of three promoters for the bacteriophage SP6 RNA polymerase' * En entier *  -/-- | 1-6 | |

**DOMAINES TECHNIQUES RECHERCHES (Int.Cl.6)**

C12N
C12Q

Le présent rapport a été établi pour toutes les revendications

| Lieu de la recherche | Date d'achèvement de la recherche | Examinateur |
|---|---|---|
| LA HAYE | 15 Mars 1995 | HORNIG H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

EP 0 659 881 A1

Office européen
des brevets

**RAPPORT DE RECHERCHE EUROPEENNE**

Numero de la demande

EP 94 40 2996

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | NUCL. ACIDS RES., vol.15, no.13, 10 Juillet 1987, IRL PRESS, OXFORD, ENGLAND; pages 5413 - 5432 K.A. CHAPMAN AND R.R. BURGESS 'Construction of bacteriophage T7 late promoters with point mutations and characterization by in vitro transcription properties' * En entier * | 1-6 | |
| D,A | J. MOL. BIOL., vol.229, 1993, ACADEMIC PRESS LIMITED, LONDON, UK; pages 805 - 811 G.A. DIAZ ET AL. 'Hirarchy of base-pair preference in the binding domain of the bacteriophage T7 promoter' * En entier * | 1-6 | |
| A | J. BIOL. CHEM., vol.267, no.16, 5 Juin 1992, AM. SOC. MOL. BIOL., INC.,BALTIMORE, US; pages 11322 - 11328 R.A. IKEDA 'The efficiency of promoter clearance distinguishes T7 class II and class III promoters' * En entier * | 1-6 | DOMAINES TECHNIQUES RECHERCHES (Int.Cl.6) |
| A | J. BIOL. CHEM., vol.267, no.4, 5 Février 1992, AM. SOC. MOL. BIOL., INC.,BALTIMORE, US; pages 2640 - 2649 R.A. IKEDA ET AL. 'Initiation of transcription by T7 RNA polymerase at its natural promoters' * En entier * | 1-6 | |

-/--

Le présent rapport a été établi pour toutes les revendications

| Lieu de la recherche | Date d'achèvement de la recherche | Examinateur |
|---|---|---|
| LA HAYE | 15 Mars 1995 | HORNIG H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

23

**Office européen des brevets**

# RAPPORT DE RECHERCHE EUROPEENNE

Numero de la demande

EP 94 40 2996

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | NUCL. ACIDS RES., vol.16, no.10, 25 Mai 1988, IRL PRESS, OXFORD, ENGLAND; pages 4511 - 4524 K.A. CHAPMAN ET AL. 'Bacteriophage T/ late promoters with point mutations: quantitative footprinting and in vivo expression' * En entier * | 1-6 | |

DOMAINES TECHNIQUES RECHERCHES (Int.Cl.6)

Le présent rapport a été établi pour toutes les revendications

| Lieu de la recherche | Date d'achèvement de la recherche | Examinateur |
|---|---|---|
| LA HAYE | 15 Mars 1995 | HORNIG H. |